# EUROPEAN PATENT APPLICATION

(11) **EP 4 012 884 A1**
(43) Date of publication of application: **15.06.2022**
(21) Application number: 20213005.0
(22) Date of filing: 10.12.2020
(51) Int. Cl.: H02J 15/00, H02J 3/28

(54) **METHOD FOR OPERATION OF AN INDUSTRIAL PLANT AND AN INDUSTRIAL PLANT**

(71) Applicant: Siemens Energy Global GmbH & Co. KG, 81739 München (DE)
(72) Inventor: Kubis, Stanislav, 66482 Ricany (CZ)

(57) **Abstract**

The present invention relates to a method for operation of an industrial plant (1) comprising an energy accumulator unit (3) for production of synthetic natural gas, a power plant unit (5) for production of electricity, an oxygen tank (7), a carbon dioxide tank (11), methanol tank (12) and a water tank (9). In a first operation mode the energy accumulator unit (3) is supplied with excessed electricity from the public grid (17) in order to produce methanol wherein the produced methanol is feed in methanol tank (12) while oxygen and water which are produced together with the methanol are stored in the oxygen tank (7) and the water tank (9) correspondingly. In a second operation mode methanol from methanol tank (12)) together with oxygen from the oxygen tank (7) and water from the water tank (9) are used in the power plant unit (5) to produce electricity, which is supplied to the public grid (17). This way electricity production excess is efficiently accumulated for industrial or municipal use.

## Description

The present invention relates to a method for operation of an industrial plant and an industrial plant Methanol Reciprocating Power plant later called MRPP

The reduction of carbon dioxide emitted into the atmosphere on the one hand and increasing the share of renewable on the other hand cause some problems. One of the problems is how to use or store the electricity excess from the renewable sources, since the amount of produced power cannot be effectively controlled. Due to this fact, there is energy generated during times when there is low or no consumption for it. This surplus of electricity is currently accumulated in different kinds of energy accumulators such as pumped water storage plant, underground pneumatic accumulators, electric accumulators, etc.

Recently, new methods of energy storage have been applied at industrial scale. The electrical energy can be accumulated or stored by converting it into chemical energy of synthetic fuels such as hydrogen, methane, methanol, or another hydrocarbon fuels. Later on, when used the term methanol it means methanol or related hydrocarbon specie which can be used as a fuel. In the prior art this process is referred to as "power to gas" technology.

One of the emerging technologies to accumulate the electricity excess is through the production of synthetic natural gas (SNG) or methanol. The technology is based on the so-called Sabatier or another chemical reaction, in which synthetic natural gas, methanol and water are formed from carbon dioxide (CO₂) and hydrogen (H₂) in the presence of catalysts. In the published studies the Carbon dioxide is obtained from biogas or coal while the hydrogen is obtained by electrolysis of water. Unfortunately, both substances biogas and coal contain certain additives. These additives play the role of impurities in the above-mentioned reactions and contaminate the catalyst. This contamination reduces the efficiency of this reactions until the reactions are not effective and the reactor has to be shutdown to recover the catalyst. It is necessary to transport these reactants for these reactions from distant sites to the synthetic fuels (like methanol) production site. Hence, there are three essential disadvantages of the above referred method: on the one hand the necessity to transport carbon dioxide or oxygen from distant locations, second the impurities in the carbon dioxide caused by the fuel additives and third energy necessary for the oxygen production.

It is object of the present invention to provide an efficient process of accumulating the electricity surplus, which process eliminates the above-identified disadvantages.

The object of the invention is achieved by the independent claims. The dependent claims describe advantageous developments and modifications of the invention.

In accordance with the invention there is provided a method for operation of an industrial plant comprising:
- an energy accumulator unit for production of synthetic methanol,
- a power plant unit for production of electricity,
- an oxygen tank, a carbon dioxide tank, methanol tank and a water tank, wherein
- in a first operation mode the energy accumulator unit is supplied with excessed electricity from the public grid in order to produce synthetic methanol, wherein the produced synthetic methanol is discharged in methanol tank, while oxygen and water which are produced together with methanol are stored in the oxygen tank and the water tank correspondingly,
- in a second operation mode methanol from the methanol tank together with oxygen from the oxygen tank and water from the water tank are used in the power plant unit to produce electricity, which is supplied to the public grid.

In accordance with the present invention there is provided also an MRPP eligible for carrying out such operation method, the MRPP comprising:
- an energy accumulator unit for production of methanol,
- a power plant unit for production of electricity,
- an oxygen tank, a carbon dioxide tank, methanol tank and a water tank.

Advantages relating to the described method may as well pertain to the MRPP and vice versa.

The essential idea of the present invention is to connect the industrial plant to the public grid which transfers easily surplus renewable energy from distant locations to the MRPP. This surplus energy is used for the production of synthetic methanol, which is then fed into the methanol tank. Along with the synthetic methanol, further oxygen and water are produced in the energy accumulator unit, which are stored locally in the MRPP.

In the second operation mode, the oxygen and water stored on site are used to produce electricity in the power plant unit of the industrial plant. The electricity generated in the power plant unit is fed into the public grid.

Thus, in the MRPP surplus energy is used to produce synthetic fuels (which is feed into a Methanol tank) and oxygen, which oxygen later is used together with synthetic fuel from methanol rank to produce energy for the public grid.

In a preferred embodiment in the first operation mode the synthetic fuels are produced in a above reactions which involves the reaction of hydrogen with carbon dioxide at elevated temperatures (for examples optimally 300-400 °C) and pressures in the presence of a catalyst to produce methane and water.

CO₂ + 3H₂ -> CH₃OH + H₂O

The chemical reaction takes part in a methanol reactor which is part of the energy accumulator unit. The chemical reaction is an important step in the creation of synthetic It can be stored in huge tanks

In another preferred embodiment water from the water tank is used in an electrolysis reaction to produce oxygen and hydrogen, which hydrogen is used for the chemical reaction. Hence, the process of electrolysis of water is carried out in a preliminary step to create hydrogen for this chemical reaction. The oxygen, which is also generated in the water electrolysis, is stored in the oxygen tank of the industrial plant.

In yet another preferred embodiment in the second operation mode the methanol is burned together with oxygen from the oxygen tank in a combustor and the combustion mixture is used to drive a turbine, in particular, a steam - gas- -turbine The combustor can be an external burner which is separated from the steam turbine or a burner integrated in the turbine. Preferably, water from the water tank is used to regulate the temperature in the combustor by spraying the water into the mixture of gas and steam coming out of the combustor.

Still preferably, the flue gas of the turbine is cooled down in a heat exchanger producing carbon dioxide, oxygen and water which are stored in the carbon dioxide, oxygen tank and the water tank correspondingly.

In order to increase the efficiency of the process running in the industrial plant, in a preferred embodiment the heat from the heat exchanger is used for district heating or applied in a condensing turbine so that a turbine with increased the power is used. (steam separation generator - Brno cycle)

Preferably, the energy accumulator unit and the power plant unit are operated alternatively. The energy accumulator unit is operated in times of electricity surplus, while the power plant unit is operated to produce supplementary electricity on demand. Alternatively, the energy accumulator unit and the power plant unit are operated simultaneously.

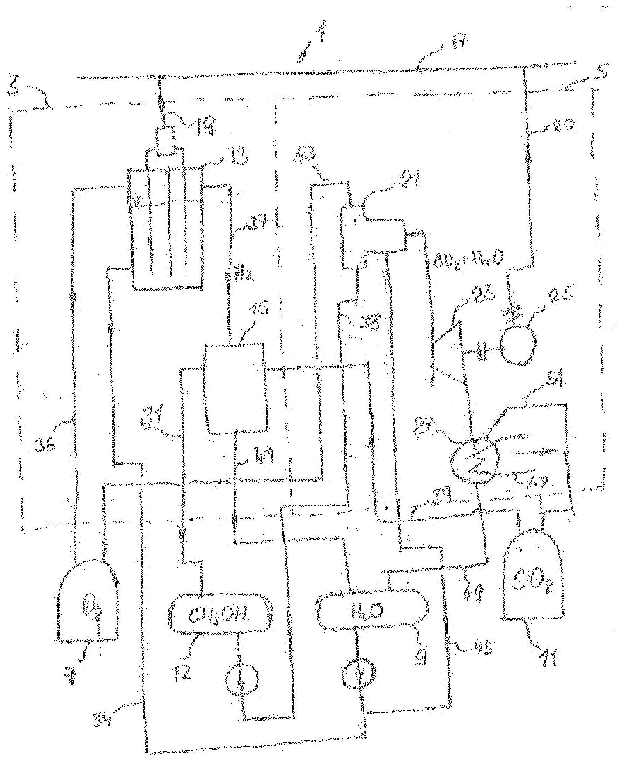

Embodiments of the invention are now described, by way of example only, with reference to the accompanying drawing, of which the only figure shows schematically an industrial plant 1. The industrial plant 1 comprises an energy accumulator unit 3 for production of synthetic fuels and a power plant unit 5 for production of electricity and heat. The industrial plant further comprises an oxygen tank 7, a water tank 9, methanol tank 12 and a carbon dioxide tank 11.

In the embodiment shown in the figure the energy accumulator unit 3 consists of a hydrogen electrolysis system 13 and a methanol reactor 15. The hydrogen electrolysis system 13 is supplied with electricity from the public grid 17 by means of a power cable 19. The methanol reactor 15 is connected to a methanol tank 12 via a gas discharge line 31.

The power plant unit 5 consists of a combustor 21, a turbine 23, a generator 25 and a heat exchanger 27. The combustor 21 is operated with synthetic fuel provided by methanol supplied line 33 from methanol tank 12.

In a first operation mode of the industrial plant 1, in case of renewable electricity surplus in the public grid 17, this excessed energy is accumulated or stored in the energy accumulator unit 3 in form of oxygen stored in the oxygen tank 7 and synthetic fuels produced in the methanol reactor 15 via the gas discharge line 31.
Water taken from the water tank 9 via a first water feed line 34 is provided to the hydrogen electrolysis system 13, in which the water molecules are split into hydrogen and oxygen using electrical current from the public grid 17. The oxygen stream is fed via an oxygen discharge line 36 into the oxygen tank 7 for storage purposes. The hydrogen stream is provided via a hydrogen feed line 37 into the methanol reactor 15, in which it reacts together with carbon dioxide provided by a feed line 39 from the carbon dioxide tank 11 to produce methanol. The methanol produced in the energy accumulator unit 3 is fed into the methanol tank 12 by means of outflow pipe 31 Another product of the chemical reaction is water, which is discharged in a first water discharge line 41 and stored in the water tank 9.

Hence, in the first operation mode excessed energy from the public grid is stored locally by converting it into oxygen and water and the remaining part of the excessed energy is stored in form of synthetic fuel(methanol) in methanol tank 12. Normally, the power plant unit 5 is shutdown during the first operation mode.

In a second operation mode methanol from the methanol tank 12 together with oxygen from the oxygen tank 7 and water from the water tank 9 are used in the MRPP unit 5 to produce electricity and heat. Methanol or another hydrocarbon fuel is supplied from the methanol tank 12 via the methanol supply line 33 into the combustor 21 to generate a steam-gas mixture. The fuel is burned there with oxygen fed from the local oxygen tank 7 via an oxygen supply line 43. The flue gas of the combustion process is a mixture of water steam and carbon dioxide and its temperature is adjusted by the amount of water from the water tank 9 via a second water feed line 45 and injected into the combustor 21. The steam-gas mixture is fed into a steam-gas turbine 23 where it expands.

The mechanical energy from the turbine 23 is transferred to the electrical generator 25, thus generating electricity. This electrical energy is conveyed to the public electricity grid 17.

The steam-gas-mixture is discharged from the turbine 23 into a heat exchanger 27 or steam separation generator. In the heat exchanger 27 the remaining thermal energy is transferred to a coolant 47 cycle. The steam gas mixture is cooled down there. The water steam from the mixture condenses and is discharged through a second water discharge line 49 into the water tank 9. The incondensable, clean carbon dioxide is discharged through a discharge line 51 into the local carbon dioxide tank 11.

This process, in which the locally stored energy (oxygen and water) together with methanol from the methanol tank are converted into electricity and heat, can be considered discharging of the energy accumulator unit 3. In this cycle, the total energy of methane, i.e. the upper heating value is employed compared with standard devices. This value is about 10% higher than the lower heating value.

Although the present invention has been described in detail with reference to the preferred embodiment, it is to be understood that the present invention is not limited by the disclosed examples, and that numerous additional modifications and variations could be made thereto by a person skilled in the art without departing from the scope of the invention.

## Claims

1. Method for operation of an MRPP industrial plant (1) comprising
- an energy accumulator unit (3) for production of synthetic natural gas,
- a power plant unit (5) for production of electricity,
- an oxygen tank (7), a carbon dioxide tank (11), a methanol tank 12 and a water tank (9),
wherein
- in a first operation mode the energy accumulator unit (3) is supplied with excessed electricity from the public grid (17) in order to produce methanol is discharged in methanol tank 12, while oxygen and water which are produced together with the methanol (synthetic hydrocarbon fuels) are stored in the oxygen tank (7) and the water tank (9) correspondingly,
- in a second operation mode methanol from the methanol tank 12 together with oxygen from the oxygen tank (7) and water from the water tank (9) are used in the power plant unit (5) to produce electricity, which is supplied to the public grid (17).

2. Method according to claim 1,
wherein in the first operation mode the methanol (synthetic hydrocarbon fuel) is produced according to chemical reaction.

3. Method according to claim 2,
wherein water from the water tank (9) is used in an electrolysis reaction to produce oxygen and hydrogen, which hydrogen is used for the chemical reaction.

4. Method according to any of the preceding claims,
wherein in the second operation mode the methanol (synthetic hydrocarbon fuel is burned together with oxygen from the oxygen tank (7) in a combustor (21) and the combustion mixture is used to drive a turbine (23), in particular a gas-steam-turbine.

5. Method according to claim 4,
wherein water from the water tank (9) is used to regulate the temperature in the combustor (21).

6. Method according to claim 5,
wherein the flue gas of the turbine (23) is cooled down in a heat exchanger (27) producing oxygen and water which are stored in the oxygen (7) tank and the water tank (9) correspondingly.

7. Method according to claim 6,
wherein the heat from the heat exchanger (27) is used for district heating or applied in a condensing turbine.

8. Method according to any of the preceding claims,
wherein the energy accumulator unit (3) and the power plant unit (5) are operated alternatingly.

9. Industrial plant (1) eligible for carrying out the method according to any of the preceding claims, comprising:
- an energy accumulator unit (3) for production of synthetic natural gas,
- a power plant unit (5) for production of electricity,
- an oxygen tank (7), a carbon dioxide (11) tank, a methanol (12) tank and a water tank (9).
